**Europäisches Patentamt**

**European Patent Office**

**Office européen des brevets**

(19)

(11) Numéro de publication : **0 453 332 B1**

(12) **FASCICULE DE BREVET EUROPEEN**

(45) Date de publication du fascicule du brevet :
02.02.94 Bulletin 94/05

(51) Int. Cl.⁵ : **B01J 27/185,** B01J 29/06,
C07C 51/377, C07C 57/04

(21) Numéro de dépôt : **91400627.5**

(22) Date de dépôt : **07.03.91**

(54) **Système catalytique résistant à l'attribution et son application à l'oxydéshydrogénation d'acides carboxyliques saturés, notamment en réacteurs à lit entraîné.**

(30) Priorité : **06.04.90 FR 9004417**

(43) Date de publication de la demande :
**23.10.91 Bulletin 91/43**

(45) Mention de la délivrance du brevet :
**02.02.94 Bulletin 94/05**

(84) Etats contractants désignés :
**AT BE CH DE DK ES FR GB GR IT LI LU NL SE**

(56) Documents cités :
**EP-A- 0 148 048**
**EP-A- 0 158 694**
**EP-A- 0 188 841**
**EP-A- 0 217 428**
**US-A- 3 862 910**
**US-A- 4 585 748**

(73) Titulaire : **ATOCHEM**
**4, Cours Michelet La Défense 10 Cédex 42**
**F-92091 Paris La Défense (FR)**

(72) Inventeur : **Dekiouk, Mohammed**
**25, rue Bellevue**
**F-5715 Woustviller (FR)**
Inventeur : **Hecquet, Gérard**
**Apt. 32, Résidence Foch 70, place Foch**
**F-62400 Bethune (FR)**
Inventeur : **Pietrzyk, Stanislas**
**6, rue Hoche**
**F-59370 Mons en Baroeul (FR)**

## Description

La présente invention porte sur un système catalytique à base d'oxydes de fer et de phosphore, résistant à l'attrition, en vue de l'oxydéshydrogénation en phase gazeuse d'acides carboxyliques saturés en acides insaturés correspondants, en particulier, en vue de l'oxydéshydrogénation de l'acide isobutyrique en acide méthacrylique, dans un réacteur à lit entraîné.

La demande de brevet européen EP-A-0 158 694 porte sur un procédé pour la conversion catalytique de l'acide isobutyrique, ou d'un ester de cet acide, en le dérivé à insaturation $\alpha,\beta$-oléfinique correspondant par oxydéshydrogénation, un catalyseur consistant en un métal alcalin ou alcalino-terreux contenant du phosphate de fer étant mis en contact avec un courant d'alimentation contenant ledit acide ou ester et de l'oxygène moléculaire à environ 300-500°C, le courant d'alimentation comprenant de l'acétone. On peut prévoir d'utiliser le catalyseur sur support (tel que silice, alumine, magnésie, oxyde de titane, quartz, carbone, carbure de silicium), et le procédé peut être conduit dans un réacteur à lit fluidisé, un réacteur à réservoir agité ou un réacteur à lit fixe ou à lit conditionné, ou toute combinaison de ces types de réacteur.

La substance à activité catalytique d'un tel système catalytique a déjà été décrite dans le brevet FR-A-2 457 795 et est représentée par la formule générale $FeP_xMe_yO_z$, dans laquelle :
- Me représente au moins l'un des éléments suivants : B, Al, Ga et In ;
- x vaut de 0,2 à 3,0 ;
- y vaut de 0,01 à 0,2 ; et
- z est la quantité d'oxygène lié aux autres éléments et correspondant à leur état d'oxydation.

Les catalyseurs de ce type se présentent, entre autres, sous une forme massique. En règle générale, la préparation s'effectue en milieu aqueux, les oxydes de fer, phosphore et du métal Me étant préparés par évaporation d'un sel de fer, d'un sel du métal Me et d'acide orthophosphorique.

Conformément au brevet FR-A-2 497 795, la réaction d'oxydation déshydrogénante est effectuée dans un réacteur à lit fixe. Le lit fixe est constitué d'une charge compacte immobile de grains de catalyseur empilés dans un tube. La taille des grains de catalyseur (quelques millimètres de diamètre) est telle qu'elle doit minimiser la perte de charge, mais elle implique ainsi une limitation diffusionnelle. Afin d'améliorer le rendement du produit désiré, la réaction est effectuée en présence de vapeur d'eau dans un rapport molaire au substrat d'environ 1 à 75, de préférence 10 à 30. Dans ces conditions, les conversions et les sélectivités sont généralement élevées, étant donné que le gaz approche l'écoulement piston et que le temps de contact peut être contrôlé avec précision. Ainsi, la conversion de l'acide isobutyrique peut atteindre jusqu'à 98%, et la sélectivité en acide méthacrylique jusqu'à 70%. Toutefois, avec une réaction catalytique exothermique, comme c'est le cas de l'oxydéshydrogénation d'acides carboxyliques saturés, il apparaît parfois des points chauds qui affectent les performances du catalyseur, notamment en modifiant les sélectivités. Il faut alors recourir à son remplacement, lorsque les performances sont affectées. De plus, la présence de fortes quantités d'eau rend très coûteuse la séparation de l'eau après réaction.

Il serait donc intéressant de pouvoir conduire de telles réactions en présence de très faibles quantités d'eau, par exemple, dans un réacteur dit à lit entraîné ou à lit transporté ou à transport pneumatique. De tels réacteurs comportent une colonne montante dans laquelle cheminent à co-courant, de bas en haut, l'alimentation gazeuse en réactifs et le catalyseur en suspension dans celle-ci. Le produit sortant au sommet de la colonne est séparé du catalyseur dans un dispositif de type cyclone. Le catalyseur est alors généralement adressé en partie haute d'une colonne de régénération à lit fluidisé, dans laquelle circule un gaz régénérant ; il sort de ladite colonne de régénération en partie basse de celle-ci, d'où il est renvoyé au bas de la colonne montante.

Un tel réacteur à lit entraîné présente les avantages suivants :
- d'une part, un écoulement piston pour le gaz et le solide, permettant un contrôle précis du temps de séjour de la masse gazeuse et de la phase solide, et
- d'autre part, il évite les courts-circuits de la zone réactionnelle par des bulles, comme cela se produit dans le cas des réacteurs à lit fluidisé,
- possibilité de réduire la teneur en eau dans la partie réactionnelle que constitue la colonne montante et de régénérer le catalyseur à l'aide d'un courant gazeux aqueux, notamment un mélange de vapeur d'eau et d'oxygène pouvant également contenir un gaz inerte comme l'azote, ainsi qu'un réactif qui compense la perte éventuelle de phosphore du catalyseur lors du cycle réactionnel,
- et enfin très faible consommation d'eau dans la partie réactionnelle.

De plus, le contrôle de la température dans le cas des réactions exothermiques est efficace, en raison du fait que les particules solides transportées évacuent la chaleur de réaction grâce à leur grande capacité calorifique. Mais surtout, l'avantage primordial est la possibilité de séparer avec précision les deux étapes du travail du catalyseur, à savoir, la réduction du catalyseur solide dans un premier temps et, dans un deuxième temps, son oxydation et sa ré-hydratation en vue de la régénération.

Pour la régénération du catalyseur, on pourra procéder conformément à l'enseignement du brevet EP-A-263 005.

Néanmoins, ce type de réacteur impose quelques contraintes : ainsi, le catalyseur doit résister à l'attrition qui résulte du choc des particules entre elles ou avec les parois internes.

La Société déposante a maintenant découvert qu'il est possible de profiter des avantages précités des réacteurs à lit entraîné pour l'oxydéshydrogénation d'acides carboxyliques saturés, grâce à un catalyseur massique du type mentionné ci-dessus, et que la mise en oeuvre de cette réaction dans un réacteur à lit entraîné peut être améliorée si ledit catalyseur est modifié pour pouvoir résister à l'attrition, par l'incorporation, dans celui-ci, d'une zéolithe.

La presente invention a donc d'abord pour objet un système catalytique comprenant le catalyseur de formule générale $FeP_xMe_yO_z$, dans laquelle Me, x, y et z sont tels que définis ci-dessus, caractérisé par le fait qu'il se présente sous une forme massique et qu'il est associé à au moins une zéolithe, la fraction constituée par la (ou les) zéolithe(s) représentant 1 à 10% en poids du catalyseur proprement dit.

Les zéolithes sont des aluminosilicates cristallisés d'origine naturelle et synthétique. Leur structure mono-, di- ou tridimensionnelle est constituée par un empilement de tétraèdres $AlO_4$ et $SiO_4$, deux tétraèdres n'étant reliés entre eux que par un seul pont oxygène. L'aluminium étant trivalent, le tétraèdre $AlO_4$ porte une charge négative compensée par un cation dont la nature est très variable ($Na^+$, $K^+$, $Ca^{++}$, $Mg^{++}$, etc., cations organiques, etc.). Les zéolithes sont représentées par la formule brute unitaire : $(M^{n+})_x$, $(AlO_2)_y^-$, $(SiO_2)_z$, $(H_2O)_w$, où M représente au moins un cation de compensation ; (n = 1 et/ou 2) ; et nx = y, y = 1, $1 \leqq z/y \leqq 6$, et $3 \leqq w \leqq 5$, et elles possèdent une structure microporeuse qui est proche de la taille des molécules. On peut choisir une zéolithe naturelle ou synthétique présentant des pores d'ouverture comprise entre, en général, 0,3 et 1,3 nm, et/ou ayant un taux de cristallinité au moins égal à 90%, comme par exemple la mordénite, et/ou possédant une granulométrie moyenne choisie notamment entre 30 et 100 $\mu$m, de préférence, entre 40 et 60 $\mu$m, et/ou présentant une surface spécifique généralement comprise entre 50 et 300 $m^2/g$, de préférence entre 100 et 200 $m^2/g$.

La surface spécifique du système catalytique selon l'invention est généralement comprise entre environ 4 et 10 $m^2/g$.

Ce système catalytique se présente notamment sous la forme de grains qui ont une dimension comprise entre environ 50 et 400 $\mu$m.

Un tel système catalytique peut être préparé par distillation à la pression atmosphérique ou sous vide (par exemple, de l'ordre de $2.10^4$ à $10^5$ Pa) d'une solution aqueuse d'acide phosphorique contenant un composé du fer (tel que $Fe(NO_3)_3 . 9 H_2O$) et un composé du métal Me, ainsi qu'une quantité allant d'environ 60 à 90% par rapport au poids de la solution aqueuse d'acide phosphorique de la zéolithe, pour éliminer une liqueur acide aqueuse, puis évaporation ou séchage, calcination, broyage et tamisage.

Ces dernières étapes sont bien connues de l'homme du métier. A titre d'exemples, on peut mentionner une évaporation à 120°C environ pendant 16-24 heures environ, ou à 180°C environ pendant 4-8 heures environ, et une calcination à environ 400-500°C, pendant environ 6-24 heures.

La présente invention a également pour objet un procédé d'oxydéshydrogénation d'acides carboxyliques saturés pour former des acides carboxyliques $\alpha,\beta$-insaturés, ce procédé consistant à mettre en contact lesdits acides saturés avec de l'oxygène moléculaire ou un gaz contenant de l'oxygène, et, le cas échéant, un gaz diluant inerte, en phase vapeur, à une température de réaction de 250 à 600°C, de préférence 350 à 450°C, en présence d'un système catalytique comprenant un catalyseur ayant la formule générale $FeP_xMe_yO_z$ dans laquelle x, y, z et Me ont des significations précitées, et modifié comme décrit précédemment, dans un réacteur à lit entraîné et, le cas échéant, en présence de vapeur d'eau dans un rapport molaire à l'acide saturé ne dépassant pas 0,5 environ.

Les conditions de travail dans la colonne montante du réacteur à lit entraîné sont généralement les suivantes : la charge envoyée dans le réacteur comprend un mélange gazeux généralement préchauffé d'acide saturé, d'oxygène moléculaire et de diluant gazeux inerte (tel que l'azote) dans lequel la proportion d'acide saturé est généralement comprise entre 1 et 8% en moles environ et le rapport molaire de l'oxygène moléculaire à l'acide saturé est compris entre 0,2 et 20, de préférence entre 0,5 et 2 environ. Lorsque de la vapeur d'eau est présente dans la charge envoyée dans le réacteur, le rapport molaire (ne dépassant pas 0,5) indiqué plus haut s'entend bien évidemment à l'exclusion de la quantité d'eau générée, comme il est bien connu, par la réaction d'oxydéshydrogénation proprement dite. Le temps de contact dans la colonne montante est de préférence choisi entre 0,1 et 50 secondes. La vitesse du gaz doit être impérativement supérieure à la vitesse limite de chute libre du catalyseur, le calcul de celle-ci étant à la portée de l'homme du métier.

La concentration volumique du système catalytique par rapport au flux gazeux est généralement comprise entre 0,1 et 15%, de préférence, entre 1 et 5% environ.

Les conditions de travail dans la colonne de régénération à lit fluidisé sont généralement les suivantes: le lit fluidisé est chargé d'une masse de catalyseur. Cette charge est mise en contact avec un flux gazeux, composé d'air, d'eau et de gaz inerte, maintenu à une température de 300°C à 500°C, destiné à contrôler l'état d'oxydo-réduction du catalyseur avant son injection dans la partie réactionnelle. La teneur volumique en eau dans le flux gazeux est généralement comprise entre 1 et 75% environ.

Les exemples suivants illustrent l'invention, sans toutefois en limiter la portée. Dans ces exemples, les pourcentages sont donnés en poids, sauf indication contraire.

EXEMPLE 1 Préparation d'un catalyseur selon l'invention de composition massique $FeP_{1,23}Cs_{0,15}$

En utilisant les quantités nécessaires, on effectue une dissolution de nitrate de césium ($CsNO_3 . H_2O$) dans de l'eau à 40°C, puis on ajoute du nitrate de fer ($Fe(NO_3)_3 . 9 H_2O$) dans la solution en agitation. On agite cette solution, à la température ambiante, pendant une nuit.

Parallèlement, on agite une solution d'$H_3PO_4$ à 85% et, par rapport au poids des oxydes du catalyseur, 3% de zéolithe Y (Al/Si = 0,524 diamètre moyen de 50 µm ; surface spécifique : 115 $m^2/g$) fournie sous la référence DA 250 (marque commerciale) par l'Institut Français du Pétrole.

On mélange les deux solutions dans un ballon avec une agitation suffisante pour homogénéiser. Ensuite, on conduit une évaporation à la pression atmosphérique et à une température de l'ordre de 115°C, avec élimination d'une solution d'acide nitrique, dont le volume est de l'ordre de 1,5 l pour un volume de départ de 8 l.

On répartit les solutions dans différents plateaux de porcelaine que l'on place à l'étuve pour une période d'une nuit à 120°C, puis à 180°C pendant 8 heures.

On calcine ensuite le gâteau à 460°C pendant 16 heures sous air ; on le concasse et on le broye pour ramener le catalyseur à une dimension adéquate (75-150 µm).

Les caractéristiques de ce catalyseur sont les suivantes :
- surface spécifique de 6 $m^2/g$
- diamètre moyen = 190 µm.

EXEMPLE 2 :

On procède comme à l'Exemple 1 pour obtenir un catalyseur de même formule massique, excepté que sa surface spécifique est de 5 $m^2/g$.

EXEMPLE COMPARATIF 3 :

On procède comme à l'Exemple 1 en remplaçant la zéolite par du tétraéthylorthosilicate, à raison de 3% par rapport à la quantité d'$H_3PO_4$ à 85%. Après évaporation, on obtient un gel. La surface spécifique du catalyseur obtenu est de 20 $m^2/g$.

EXEMPLE COMPARATIF 4 :

On procède comme à l'Exemple 1 en utilisant 90% de la zéolite par rapport à la quantité d'$H_3PO_4$ à 85%.

EXEMPLE COMPARATIF 5 :

On procède comme à l'Exemple 1, excepté que l'on remplace la zéolite par la même quantité de silice colloïdale (LUDOX (marque déposée)), et qu'après l'évaporation à 120°C pendant une nuit et à 180°C pendant 8 heures, on conduit un broyage, un pastillage avec du carbone, puis une calcination à 460°C pendant 16 heures.

La surface spécifique de ce catalyseur est de 5 $m^2/g$.

EXEMPLE 6 Estimation de l'attrition

On a effectué sur les catalyseurs des Exemples i et 2 et Comparatif 3 à 5, le test d'attrition commercialisé par la Société Géomécanique, à Rueil-Malmaison.

Les vitesses d'attrition sont basées sur la production de particules de fines inférieures à une dimension (d) donnée pendant 1 heure et sont définies par la formule :

$$\frac{\% \text{ final des fines} < d \ - \ \% \text{ initial} < d}{\% \text{ initial de fine} < d} \times 100 \ h^{-1}$$

**4**

Les résultats obtenus sont regroupés dans le Tableau I suivant pour les fractions inférieures à respectivement 50 µm et 90 µm.

## TABLEAU I

| Exemple | d < 50 µm | d < 90 µm |
|---------|-----------|-----------|
| 1 | 3,6 | 2,5 |
| Comp. 2 | 15,9 | 10,1 |
| Comp. 3 | 13,0 | n.d. |
| Comp. 4 | 10,4 | 6,7 |

n.d. = non déterminé

Il ressort à l'évidence du Tableau I que le catalyseur ayant la meilleure tenue à l'attrition est celui de l'Exemple 1 selon l'invention.

## EXEMPLE 7 à 12

Réaction de déshydrogénation de l'acide isobutyrique (AIB) en acide méthacrylique dans un réacteur à lit entraîné

Le réacteur comprend une colonne montante chauffée (ou section réactionnelle) en acier inoxydable, d'un diamètre intérieur de 20 mm pour 3 mm d'épaisseur, et d'une hauteur de 2,8 m. La séparation gaz - solide à la sortie de ladite colonne montante est réalisée dans un cyclone. La colonne de régénération comporte un lit fluidisé chauffé, dont le rapport hauteur/diamètre est fixé à 2.

On injecte l'acide isobutyrique liquide dans un mélange oxygène/azote dans un vaporiseur rempli de garnissage de quartz pour homogénéiser et vaporiser à une température inférieure à 350°C pour éviter la combustion de l'acide isobutyrique. La teneur désirée en oxygène dans le mélange gazeux est obtenue par dilution de l'air dans l'azote, chacun des débits étant contrôlé par des débitmètres massiques.

On injecte le mélange ci-dessus à 350°C environ dans la colonne montante qui est maintenue à une température de 415°C. Dans cette colonne, le mélange chemine de bas en haut à co-courant avec le système catalytique de l'Exemple 1, lequel provient du lit fluidisé.

Dans le Tableau II ci-après, figurent, pour chacun de ces exemples, le débit total du flux gazeux de la colonne montante, le pourcentage molaire de l'acide isobutyrique et le rapport molaire $O_2$/acide isobutyrique dans la colonne montante.

On adresse le flux sortant de la colonne montante à un cyclone, en vue de séparer le système catalytique que l'on régénère. A cet effet, on charge le lit fluidisé avec une masse de ce catalyseur, laquelle est mise en contact avec un flux gazeux, maintenu à une température de 450°C, composé d'air, d'eau et d'azote, auquel on a ajouté 100 ppm de triéthylphosphate pour compenser la perte éventuelle de phosphore du catalyseur lors du cycle réactionnel. Le débit total de ce flux gazeux et le débit d'eau figurent également dans le Tableau II.

En opérant dans les conditions qui viennent d'être indiquées, on obtient une bonne conversion de l'acide isobutyrique et une bonne sélectivité en acide méthacrylique.

## TABLEAU II

| Exemple | Débit Total colonne montante (mole/h) | % molaire d'AIB dans colonne montante | Rapport molaire $O_2$/AIB dans la colonne montante | Débit total flui-disation (mole/h) | Débit d'eau (mole/h) dans la fluidisation (régénération) |
|---|---|---|---|---|---|
| 7 | 83 | 5,37 | 1,26 | 124,3 | 20,6 |
| 8 | 85 | 1,91 | 0,82 | 109,3 | 0 |
| 9 | 85 | 1,91 | 0,82 | 70,9 | 0 |
| 10 | 85 | 1,91 | 0,82 | 117,10 | 8,1 |
| 11 | 103,6 | 4,93 | 0,3 | 85,6 | 0 |
| 12 | 103,6 | 4,93 | 0,3 | 168,3 | 82,8 |

EP 0 453 332 B1

**Revendications**

1. Système catalytique comprenant le catalyseur représenté par la formule générale $FeP_xMe_yO_z$, dans laquelle :
   - Me représente au moins l'un des éléments suivants : Li, Na, K, Rb, Cs, Mg, Ca, Sr et Ba ;
   - x vaut de 0,2 à 3,0 ;
   - y vaut de 0,01 à 0,2 ; et
   - z est la quantité d'oxygène lié aux autres éléments et correspondant à leur état d'oxydation,
   caractérisé par le fait qu'il se présente sous une forme massique et qu'il est associé à au moins une zéolithe, la fraction constituée par la (ou les) zéolithe(s) représentant 1 à 10% en poids du catalyseur proprement dit.

2. Système catalytique selon la revendication 1, caractérisé par le fait que la zéolithe utilisée présente des pores d'ouverture comprise entre 0,3 et 1,3 nm, et/ou un taux de cristallinité au moins égal à 90%, et/ou une granulométrie moyenne de 30 à 100 μm, et/ou une surface spécifique comprise entre 50 et 300 m²/g.

3. Système catalytique selon l'une des revendications 1 et 2, caractérisé par le fait que sa surface spécifique est comprise entre 4 et 10 m²/g.

4. Système catalytique selon l'une des revendications 1 à 3, caractérisé par le fait qu'il se présente sous la forme de grains qui ont une dimension comprise entre 50 et 400 μm.

5. Procédé d'oxydéshydrogénation d'acides carboxyliques saturés pour former des acides carboxyliques α,β-insaturés, ce procédé consistant à mettre en contact lesdits acides saturés avec de l'oxygène moléculaire ou un gaz contenant de l'oxygène, et, le cas échéant, un gaz diluant inerte, en phase vapeur, à une température de réaction de 250 à 600°C, en présence d'un système catalytique tel que défini à l'une des revendications 1 à 4, dans un réacteur à lit entraîné et, le cas échéant, en présence de vapeur d'eau dans un rapport molaire à l'acide saturé ne dépassant pas 0,5.

6. Procédé selon la revendication 5, caractérisé par le fait que l'on envoie dans la colonne montante du réacteur à lit entraîné un mélange gazeux généralement préchauffé d'acide saturé, d'oxygène moléculaire et de diluant gazeux inerte, dans lequel la proportion d'acide saturé est comprise entre 1 et 8% en moles et le rapport molaire de l'oxygène moléculaire à l'acide saturé est compris entre 0,2 et 20, et que le temps de contact dans la colonne montante est choisi entre 0,1 et 50 secondes.

7. Procédé selon l'une des revendications 5 et 6, caractérisé par le fait que la concentration volumique du système catalytique par rapport au flux gazeux dans la colonne montante est comprise entre 0,1 et 15%.

8. Procédé selon l'une des revendications 5 à 7, caractérisé par le fait que, pour régénérer le catalyseur, on charge une colonne de régénération à lit fluidisé de la masse catalytique à régénérer, et on met cette charge en contact avec un flux gazeux composé d'air, d'eau et de gaz inerte maintenu à une température de 300°C à 500°C, la teneur volumique en eau dans le flux gazeux étant comprise entre 1 et 75%.

**Patentansprüche**

1. Katalytisches System mit dem Katalysator der allgemeinen Formel $FeP_xMe_yO_z$, in der
   - Me mindestens eines der folgenden Elemente darstellt: Li, Na, K, Rb, Cs, Mg, Ca, Sr und Ba,
   - x einen Wert von 0,2 bis 3,0 hat,
   - y einen Wert von 0,01 bis 0,2 hat, und
   - z die an die anderen Elemente gebundene und deren Oxidationszustand entsprechende Sauerstoffmenge ist,
   dadurch gekennzeichnet, daß es in Form einer Masse und im Verband mit mindestens einem Zeolith vorliegt, wobei die von dem (oder den) Zeolith(en) gebildete Fraktion 1 bis 10 Gew.-% des eigentlichen Katalysators ausmacht.

2. Katalytisches System nach Anspruch 1, dadurch gekennzeichnet, daß der verwendete Zeolith Poren einer Weite zwischen 0,3 und 1,3 nm und/oder einen Kristallinitätsgrad von mindestens 90% und/oder eine mittlere Korngröße von 30 bis 100 μm und/oder eine spezifische Oberfläche zwischen 50 und 300 m²/g aufweist.

3. Katalytisches System nach Anspruch 1 oder 2, dadurch gekennzeichnet, daß seine spezifische Oberfläche zwischen 4 und 10 m²/g beträgt.

4. Katalytisches System nach einem der Ansprüche 1 bis 3, dadurch gekennzeichnet, daß es in der Form von Körnern vorliegt, die eine Größe zwischen 50 und 400 μm besitzen.

5. Verfahren zur Oxydehydrierung von gesättigten Karbonsäuren, um α,β-ungesättigte Karbonsäuren zu bilden, wobei das Verfahren darin besteht, die genannten gesättigten Karbonsäuren mit molekularem Sauerstoff oder einem sauerstoffhältigen Gas und gegebenenfalls mit einem inerten Verdünnungsgas in der Dampfphase bei einer Reaktionstemperatur von 250° bis 600°C in Gegenwart eines katalytischen Systems, wie es in einem der Ansprüche 1 bis 4 definiert ist, in einem Wirbelbettreaktor und gegebenenfalls in Gegenwart von Wasserdampf in einem 0,5 nicht überschreitenden Molverhältnis in bezug auf die gesättigte Säure in Kontakt zu bringen.

6. Verfahren nach Anspruch 5, dadurch gekennzeichnet, daß man in die ansteigende Kolonne des Wirbelbettreaktors ein im allgemeinen vorerhitztes gasförmiges Gemisch der gesättigten Säure, des molekularen Sauerstoffs und des inerten Verdünnungsgases einleitet, in welchem Gemisch der Prozentsatz der gesättigten Säure zwischen 1 und 8 Mol-% beträgt und das Molverhältnis des molekularen Sauerstoffs zur gesättigten Säure zwischen 0,2 und 20 liegt, und daß die Dauer des Kontaktes in der ansteigenden Kolonne zwischen 0,1 und 50 Sekunden gewählt wird.

7. Verfahren nach Anspruch 5 oder 6, dadurch gekennzeichnet, daß die Volumenkonzentration des katalytischen Systems in bezug auf den Gasstrom in der ansteigenden Kolonne zwischen 0,1 und 15% beträgt.

8. Verfahren nach einem der Ansprüche 5 bis 7, dadurch gekennzeichnet, daß man, um den Katalysator zu regenerieren, eine Fließbett-Regenerationskolonne mit der zu regenerierenden katalytischen Masse beschickt und diese Charge mit einem aus Luft, Wasser und inertem Gas zusammengesetzten und auf einer Temperatur von 300° bis 500°C gehaltenen gasförmigen Strom in Kontakt bringt, wobei der Volumengehalt des gasförmigen Stromes an Wasser zwischen 1 und 75% beträgt.

## Claims

1. Catalyst system comprising the catalyst denoted by the general formula FeP$_x$Ne$_y$O$_z$, in which:
   - Me denotes at least one of the following elements: Li, Na, K, Rb, Cs, Mg, Ca, Sr and Ba,
   - x has a value from 0.2 to 3.0,
   - y has a value from 0.01 to 0.2, and
   - z is the quantity of oxygen bonded to the other elements and corresponding to their oxidation state, characterized in that it is in a bulk solid form and that it is associated with at least one zeolite, the fraction formed by the zeolite(s) representing 1 to 10 % by weight of the actual catalyst.

2. Catalyst system according to Claim 1, characterized in that the zeolite employed has pores with an opening of between 0.3 and 1.3 nm, and/or a crystallinity of at least 90 % and/or a mean particle size of 30 to 100 μm and/or a specific surface of between 50 and 300 m²/g.

3. Catalyst system according to either of Claims 1 and 2, characterized in that its specific surface is betweem 4 and 10 m²/g.

4. Catalyst system according to one of Claims 1 to 3, characterized in that it takes the form of particles which have a dimension of between 50 and 400 μm.

5. Process for oxidative dehydrogenation of saturated carboxylic acids to form α,β-unsaturated carboxylic acids, this process consisting in bringing the said saturated acids into contact with molecular oxygen or a gas containing oxygen and, where appropriate, an inert diluent gas, in vapour phase, at a reaction temperature of 250 to 600°C, in the presence of a catalyst system as defined in one of Claims 1 to 4, in an entrained-bed reactor and, where appropriate, in the presence of steam in a molar ratio to the saturated acid not exceeding 0.5.

6. Process according to Claim 5, characterized by sending into the upward column of the entrained-bed reactor a generally preheated gaseous mixture of saturated acid, molecular oxygen and inert gaseous diluent, in which the proportion of saturated acid is between 1 and 8 mol% and the molar ratio of molecular oxygen to the saturated acid is between 0.2 and 20 and the contact time in the upward column is chosen between 0.1 and 50 seconds.

7. Process according to either of Claims 5 and 6, characterized in that the volume concentration of the catalyst system in relation to the gas stream in the upward column is between 0.1 and 15 %.

8. Process according to one of Claims 5 to 7, characterized in that, to regenerate the catalyst, the fluidized-bed regenerating column is charged with the catalytic mass to be regenerated and this charge is brought into contact with a gas stream made up of air, water and inert gas, maintained at a temperature of 300°C to 500°C, the volume content of water in the gas stream being between 1 and 75 %.